# EUROPEAN PATENT SPECIFICATION

(11) **EP 1 471 907 B1**
(45) Date of publication and mention of the grant of the patent: **16.07.2008**
(21) Application number: 02765159.5
(22) Date of filing: 28.06.2002
(51) Int. Cl.: A61K 31/506, A61P 17/00, A61P 37/00, A61P 1/16

(54) **USE OF C-KIT INHIBITORS FOR TREATING AUTOIMMUNE DISEASES**
DIE VERWENDUNG VON C-KITHEMMERN ZUR BEHANDLUNG VON AUTOIMMUNERKRANKUNGEN
UTILISATION D'INHIBITEURS DE C-KIT DESTINES A TRAITER DES MALADIES AUTO-IMMUNES

(30) Priority: 29.06.2001 US 301405 P; 29.06.2001 US 301410 P; 29.06.2001 US 301409 P; 20.12.2001 US 341273 P
(43) Date of publication of application: 03.11.2004
(73) Proprietor: AB Science, 75008 Paris (FR)
(72) Inventor: MOUSSY, Alain, F-75006 Paris (FR); KINET, Jean-Pierre, Lexington, MA 02173 (US)
(74) Representative: Warcoin, Jacques
(86) International application number: PCT/IB2002/003302
(87) International publication number: WO 2003/002109

(56) References cited:
- EP-A- 0 564 409
- WO-A-01/47507
- WO-A-01/64200
- WO-A-02/22597
- WO-A-02/080925
- GILBERT RICHARD E ET AL: "PDGF signal transduction inhibition ameliorates experimental mesangial proliferative glomerulonephritis" KIDNEY INTERNATIONAL, vol. 59, no. 4, April 2001 (2001-04), pages 1324-1332, XP002327722 ISSN: 0085-2538
- SIHVOLA R ET AL: "Prevention of cardiac allograft arteriosclerosis by protein tyrosine kinase inhibitor selective for platelet-derived growth factor receptor." CIRCULATION. 4 MAY 1999, vol. 99, no. 17, 4 May 1999 (1999-05-04), pages 2295-2301, XP008046678 ISSN: 1524-4539
- KALLIO E A ET AL: "Role of platelet-derived growth factor in obliterative bronchiolitis (chronic rejection) in the rat" AMERICAN JOURNAL OF RESPIRATORY AND CRITICAL CARE MEDICINE 1999 UNITED STATES, vol. 160, no. 4, 1999, pages 1324-1332, XP008046679 ISSN: 1073-449X
- GAMBACORTI-PASSERINI C ET AL: "ROLE OF ALPHA1 ACID GLYCOPROTEIN IN THE IN VIVO RESISTANCE OF HUMAN BCRABL+ LEUKEMIC CELLS TO THE ABL INHIBITOR ST1571" JOURNAL OF THE NATIONAL CANCER INSTITUTE, US DEPT. OF HEALTH, EDICATIONAND WELFARE, PUBLIC HEALTH, US, vol. 92, no. 20, 18 October 2000 (2000-10-18), pages 1641-1650, XP001036595 ISSN: 0027-8874
- HEINRICH MICHAEL C ET AL: "Inhibition of c-kit receptor tyrosine kinase activity by STI 571, a selective tyrosine kinase inhibitor" BLOOD, vol. 96, no. 3, 1 August 2000 (2000-08-01), pages 925-932, XP001097629 ISSN: 0006-4971
- CARROLL M ET AL: "CGP 57148, a tyrosine kinase inhibitor, inhibits the growth of cells expressing BCR-ABL, TEL-ABL, and TEL-PDGFR fusion proteins." BLOOD. 15 DEC 1997, vol. 90, no. 12, 15 December 1997 (1997-12-15), pages 4947-4952, XP002327723 ISSN: 0006-4971
- NICOLAUS B.J.R.: "Symbiotc approach to drug design. Decision making in drug research" 1983, RAVEN PRESS, EDITEDBY FRANZ GROSS , NEW YORK, USA * page 173 - page 186 *
- BROUARD, M.C., ET. AL.: "Acute generalized exanthematous pustulosis associated with STI 571 in a patient with chronic myeloid leukemia" DERMATOLOGY, vol. 203, no. 1, 2001, pages 57-59,
- BROUARD, M., ET. AL.: "Cutaneous reactions to STI571" NEW ENGLAND JOURNAL OF MEDICINE, vol. 345, no. 8, 2001, pages 618-619,
- HAMM, M., ET. AL.: "Imatinib-induced purpuric vasculitis" ANNALES DE DERMATOLOGIE ET DE VENEREOLOGIE, vol. 130, no. 8-9, 2003, pages 765-767,
- PUXEDDU, I., ET. AL.: "Cells in focus: Mast cells in allergy and beyond." INTERNATIONAL JOURNAL OF BIOCHEMISTRY & CELL BIOLOGY, vol. 35, 2003, pages 1601-1607,
- DING-ZHU SHEN: "A target rle for mast cell in the prevention and therapy of hepatic fibrosis" MEDICAL HYPOTHESES, 2007,
- GHALY, N.R., ET AL.: "Role of mast cells and T-lymphocytes in phemphigus vulgaris: significance of CD44 and the c-kit gene product (CD117)." EAST MEDITERR. HEALTH, vol. 11, no. 5-6, 2005, pages 1009-1017,
- EKLUND, K.K., ET. AL.: "Mast cells in the pathogenesis of rheumatic diseases and as potential targets for anti-rheumatic therapy" IMMUNOLOGICAL REVIEWS, vol. 217, 2007, pages 38-52,
- ISHII, M., ET. AL.: "A role of mast cells for hepatic fibrosis in primary sclerosing cholangitis" HEPATOLOGY RESEARCH, vol. 31, no. 3, 2005, pages 127-131,

### SEQUENCE LISTING

<110> AB Science
<120> Use of tyrosine kinase inhibitors for treating autoimmune diseases
<130> 342884 NT
<150> US 60/341,273
   <151> 2001-12-20
<150> US 60/301,405
   <151> 2001-06-29
<150> US 60/301,409
   <151> 2001-06-29
<150> US 60/301,410
   <151> 2001-06-29
<160> 5
<170> PatentIn Ver. 2.1
<210> 1
   <211> 976
   <212> PRT
   <213> Homo sapiens
<220>
   <223> Human c-kit
<400> 1
<210> 2
   <211> 30
   <212> DNA
   <213> Homo sapiens
<220>
   <223> Primer
<400> 2
   aagaagagat ggtacctcga ggggtgaccc 30
<210> 3
   <211> 33
   <212> DNA
   <213> Homo sapiens
<220>
   <223> Primer
<400> 3
   ctgcttcgcg gccgcgttaa ctcttctcaa cca 33
<210> 4
   <211> 20
   <212> DNA
   <213> Homo sapiens
<220>
   <223> Primer
<400> 4
   agctcgttta gtgaaccgtc 20
<210> 5
   <211> 20
   <212> DNA
   <213> Homo sapiens
<220>
   <223> Primer
<400> 5
   gtcagacaaa atgatgcaac 20

## Description

The present invention relates to the use of a c-kit inhibitor chosen from N-phenyl-2-pyrimidine-amines for preparing a medicament for treating autoimmune diseases selected from the group consisting of subepidermal blistering disorders, systemic lupus erythematosus, discoid lupus erythematosus, cutaneous lupus, dermatomyositis, polymyositis, Sjogren's syndrome, primary biliary cirrhosis, active chronic hepatitis, chronic fatigue syndrome and Vasculitis.

Autoimmune diseases arise when immune system cells (lymphocytes, macrophages) become sensitized against the "self". Lymphocytes TH and CTL, B lymphocytes as well as macrophages are usually under control in this system. But, a misdirection of the system toward the body's own tissues may happen for still unexplained triggers. In other words, autoimmune disorders occur when the normal control process is disrupted.

The hypothesis is that lymphocytes recognize at some point an antigen which mimics the "self" and a cascade of activation of different components of the immune system takes place, ultimately leading to tissue destruction. Genetic predisposition has also been postulated to be responsible for autoimmune disorders. Autoimmune diseases can affect connective tissue, but it can also affect the nerves, muscles, endocrine system, and the gastrointestinal system.

According to the American Autoimmune Related Diseases Association (AARDA), autoimmune diseases must be regarded "as a united group of disorders". Indeed, the presence of one autoimmune disease in one patient implies the possibility that a second or third autoimmune disease may occur in the same individual or in other members of the same family.

As suggested by the AARDA, an effective treatment of autoimmune disease requires the identification and turning off these disease-producing T cells. But as of today, such a cure remain elusive.

Typically, these diseases are treated with corticosteroids and immunosuppressant medications (including cyclophosphamide or azathioprine) to reduce the immune response. In addition, US 6,248,368 describes the use of magnesium to treat autoimmune diseases in association with vitamin B6. Compositions containing purified anti-idiotypic antibodies have also been proposed in US 6,231,856 and compositions containing an antisense oligonucleotides targeted to nucleic acids encoding TNFα are mentioned in US 6,228,642 for treating autoimmune diseases. Alternatively, it has been suggested in WO9841525 to treat autoimmune diseases with pyrrolo[2,3D]pyrimidines.

But, none of the above available treatments are effective and safe for treating autoimmune diseases. In addition, the prolonged use of immunosuppressor drugs lead to adverse side effects and morbidity. Moreover, autoimmunity related disorders require lifetime care and treatment, which is expensive and often lead to the disruption of the lifestyle of patients.

Therefore, the problem is to find alternative solutions to provide a relief and a cure for the numerous patients afflicted with these diseases.

In connection with the present invention, we propose that mast cells are the central players involved in autoimmune diseases. Mast cells (MC) are tissue elements derived from a particular subset of hematopoietic stem cells that express CD34, c-kit and CD13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997).

Activation of the detrimental immune response to the self is postulated here to results or to be further stimulated from the degranulation of mast cells. Among to cytokines secreted by mast cells, IFNγ is of particular interest. Indeed, it has been observed that IFNγ is responsible for major histocompatibility complex (MHC) associated autoimmune diseases; Hooks et al, (1979) New England J.Med., Vol. 301 : 5-8. For example, higher IFNγ levels were shown to correlate with greater severity of disease in SLE patients.

TNF is another cytokine produced by mast cells. More recently, it has been reported that TNF produced by mast cells was involved in the pathogenesis of autoantibody-mediated vasculitis, Watanabe N. et al Blood 1999 Dec 1;94(11):3855-63. In Biedermann T et al, J Exp Med 2000 Nov 20;192(10):1441-52, it is shown that mast cells control neutrophil recruitment during T cell-mediated delayed-type hypersensitivity reactions through TNF and macrophage inflammatory protein 2 (MIP-2).

In addition, mast cells are postulated here to participate in the destruction of tissues by releasing a cocktail of different proteases and mediators categorized into three groups: preformed granule-associated mediators (histamine, proteoglycans, and neutral proteases), lipid-derived mediators (prostaglandins, thromboxanes and leucotrienes), and various cytokines (IL-1, IL-2, IL-3, IL-4, IL-5, IL-6, IL-8, TNF-α, GM-CSF, MIP-la, MIP-1b, MIP-2 and IFN-γ). Then, liberation by activated mast cells of mediators (TNF-α, histamine, leucotrienes, prostaglandines etc...) as well as proteases is proposed here i) to induce and activate components of the immunity involved in autoimmune diseases and ii) to participate in the tissue destruction process. The activation of T and B lymphocytes against the self stimulate mast cells, which in turn release the above mentioned factors further activating components of the autoimmune reaction.

To break the formation of this cycle leading to tissue destruction, the present invention proposes to deplete mast cells using compounds that are substantially specific to mast cells. In this regard, c-kit specific kinase inhibitors are proposed to inhibit mast cell proliferation, survival and activation.

A new route for treating autoimmune diseases is provided, which consists of destroying mast cells playing a role in the pathogenesis of these disorders. It has been found that c-kit inhibitors are especially suited to reach this goal.

### Description

The present invention relates to the use of N-phenyl-2-pyrimidine-amines having the formula II :

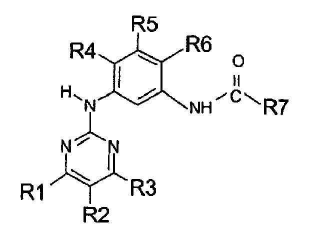

Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group;
for preparing a medicament for treating autoimmune diseases selected from the group consisting of subepidermal blistering disorders, systemic lupus erythematosus, discoid lupus erythematosus, cutaneous lupus, dermatomyositis, polymyositis, Sjogren's syndrome, primary biliary cirrhosis, active chronic hepatitis, chronic fatigue syndrome and Vasculitis.

Preferably, R7 is

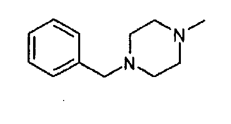

Said inhibitor is the 4-(4-methylpiperazine-1-ylmethyl)-N-[4-methyl-3-(4-pyridine-3-yl)pyrimidine-2 ylamino)phenyl]-benzamide corresponding to the following formula :

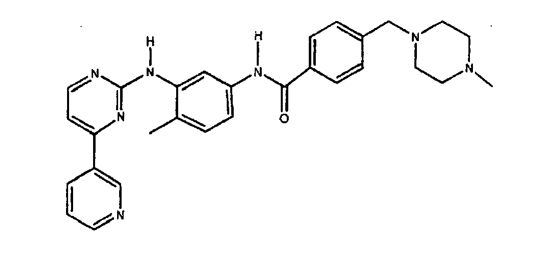

The preparation of this compound is described in example 21 of EP 564 409 and the β-form, which is particularly useful is described in WO 99/03854.

The invention also relates to the use of a composition suitable for topical administration comprising a c-kit inhibitor as defined above for the treatment systemic lupus erythematosus, discoid lupus erythematosus, cutaneous lupus, dermatomyositis and Vasculitis.

It is also aimed at the use of a c-kit inhibitor as defined above combined with at least one antibiotic, preferably selected from dapsone, azathioprine, erythromycin, propionylerythromycin, neomycin, gentomycin, tobramycin, and mechlocycline for preparing a medicament for simultaneous, separate or sequential use for the treatment of subepidermal blistering disorders, such as pemphigus.

As indicated above, the invention is applicable to the treatment of subepidermal blistering disorders. The following subepidermal blistering disorders as referred herein are contemplated by the present invention : aphthous ulcers, and several bullous diseases such as pemphigus, bullous pemphigoid and cicatricial pemphigoid.

The invention as depicted above is particularly useful for treating Pemphigus vulgaris. In this disorder, lesions occur in the mouth, as well as on the chest, scalp, periumbilical, and various other areas of the skin. Oral lesions have also been observed. This form of the disease can involve the oropharynx and other mucosal surfaces ; this why the invention contemplates compositions for topical as well as oral administration suitable to reach the particular tissues indicated above.

The invention as depicted above is particularly useful for treating Pemphigus vegetans, in which vegetating legions and pustules form. Pustules are the result of a superinfection at the edges of the broken bullae. In this regard, antibiotics can be used concomitant with c-kit inhibitors. Among antibiotics, the preferred ones are selected from dapsone, azathioprine, erythromycin, propionylerythromycin, neomycin, gentomycin, tobramycin, and mechlocycline. At last, hyperkeratosis, pseudoepitheliomatous hyperplasia, and papillomatosis have also been observed in this disease and will be treated accordingly.

The invention as depicted above is also particularly useful for treating Pemphigus foliaceus, which symptoms include crusting, scales, erosion, and excoriations.

The invention as depicted above is also particularly useful for treating Pemphigus erythematosus. Here, lesions are lupus-like butterfly rash as well as bullous and seborrheic dermatitis-like lesions.

The invention as depicted above is also particularly useful for treating Vasculitis which involves inflammation in blood vessels of various sizes from the aorta to the smallest blood vessels in the skin. This group of diseases encompasses Giant Cell Arteritis, Polymyalgia Rheumatica, Wegener's Granulomatosis, Polyarteritis Nodosa, Hypersensitivity Vasculitis, Rheumatoid Vasculitis, Microscopic Polyangiitis, Buerger's Disease Kawasaki's Disease as well as Vasculitis caused by infection or allergy.

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

More particularly, the invention relates to a pharmaceutical composition intended for oral or topical administration.

Regarding topical administration, the compositions according to the invention may be presented in the form of a gel, paste, ointment, cream, lotion, liquid suspension aqueous, aqueous-alcoholic or, oily solutions, or dispersions of the lotion or serum type, or anhydrous or lipophilic gels, or emulsions of liquid or semi-solid consistency of the milk type, obtained by dispersing a fatty phase in an aqueous phase or vice versa, or of suspensions or emulsions of soft, semi-solid consistency of the cream or gel type, or alternatively of microemulsions, of microcapsules, of microparticles or of vesicular dispersions to the ionic and/or nonionic type. These compositions are prepared according to standard methods.

The composition according to the invention comprises any ingredient commonly used in dermatology and cosmetic. It may comprise at least one ingredient selected from hydrophilic or lipophilic gelling agents, hydrophilic or lipophilic active agents, preservatives, emollients, viscosity enhancing polymers, humectants, surfactants, preservatives, antioxidants, solvents, and fillers, antioxidants, solvents, perfumes, fillers, screening agents, bactericides, odor absorbers and coloring matter.

As oils which can be used in the invention, mineral oils (liquid paraffin), vegetable oils (liquid fraction of shea butter, sunflower oil), animal oils, synthetic oils, silicone oils (cyclomethicone) and fluorinated oils may be mentioned. Fatty alcohols, fatty acids (stearic acid) and waxes (paraffin, carnauba, beeswax) may also be used as fatty substances.

As emulsifiers which can be used in the invention, glycerol stearate, polysorbate 60 and the PEG-6/PEG-32/glycol stearate mixture are contemplated.
As hydrophilic gelling agents, carboxyvinyl polymers (carbomer), acrylic copolymers such as acrylate/alkylacrylate copolymers, polyacrylamides, polysaccharides such as hydroxypropylcellulose, clays and natural gums may be mentioned, and as lipophilic gelling agents, modified clays such as bentones, metal salts of fatty acids such as aluminum stearates and hydrophobic silica, or alternatively ethylcellulose and polyethylene may be mentioned.

As hydrophilic active agents, proteins or protein hydrolysates, amino acids, polyols, urea, allantoin, sugars and sugar derivatives, vitamins, starch and plant extracts, in particular those of Aloe vera may be used.

As lipophilic active, agents, retinol (vitamin A) and its derivatives, tocopherol (vitamin E) and its derivatives, essential fatty acids, ceramides and essential oils may be used. These agents add extra moisturizing or skin softening features when utilized.

In addition, a surfactant can be included in the composition so as to provide deeper penetration of the c-kit inhibiting compound capable of depleting mast cells.

Among the contemplated ingredients, the invention embraces penetration enhancing agents selected for example from the group consisting of mineral oil, water, ethanol, triacetin, glycerin and propylene glycol; cohesion agents selected for example from the group consisting of polyisobutylene, polyvinyl acetate and polyvinyl alcohol, and thickening agents.

Chemical methods of enhancing topical absorption of drugs are well known in the art. For example, compounds with penetration enhancing properties include sodium lauryl sulfate (Dugard, P. H. and Sheuplein, R. J., "Effects of Ionic Surfactants on the Permeability of Human Epidermis: An Electrometric Study," J. Ivest. Dermatol., V.60, pp. 263-69, 1973), lauryl amine oxide (Johnson et. al., US 4,411,893), azone (Rajadhyaksha, US 4,405,616 and 3,989,816) and decylmethyl sulfoxide (Sekura, D. L. and Scala, J., "The Percutaneous Absorption of Alkylmethyl Sulfides," Pharmacology of the Skin, Advances In Biolocy of Skin, (Appleton-Century Craft) V. 12, pp. 257-69, 1972). It has been observed that increasing the polarity of the head group in amphoteric molecules increases their penetration-enhancing properties but at the expense of increasing their skin irritating properties (Cooper, E. R. and Berner, B., "Interaction of Surfactants with Epidermal Tissues: Physiochemical Aspects," Surfactant Science Series, V. 16, Reiger, M. M. ed. (Marcel Dekker, Inc.) pp. 195-210, 1987).

A second class of chemical enhancers are generally referred to as co-solvents. These materials are absorbed topically relatively easily, and, by a variety of mechanisms, achieve permeation enhancement for some drugs. Ethanol (Gale et. al., U.S. Pat. No. 4,615,699 and Campbell et. al., U.S. Pat. Nos. 4,460,372 and 4,379,454), dimethyl sulfoxide (US 3,740,420 and 3,743,727, and US 4,575,515), and glycerine derivatives (US 4,322,433) are a few examples of compounds which have shown an ability to enhance the absorption of various compounds.

Topical compositions referred herein are also particularly relevant for treating aphthous ulcers, and several bullous diseases such as pemphigus, bullous pemphigoid and cicatricial pemphigoid since they are affecting especially the skin and mucosal membranes.

Pharmaceutical compositions suitable for use in the invention include compositions wherein the c-kit inhibiting compounds for depleting mast cells are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. As mentioned above, a c-kit inhibitor according to the invention is unable to promote death of IL-3 dependent cells cultured in presence of IL-3.

The invention also contemplates a product comprising a c-kit inhibitor as defined above and at least one antibiotic, the preferred ones being selected from cyclophosphamide, methotrexate, dapsone, azathioprine, erythromycin, propionylerythromycin, neomycin, gentomycin, tobramycin, and mechlocycline for simultaneous, separate or sequential use for the treatment of subepidermal blistering disorders, such as pemphigus.

Utility of the invention will further ensue from the detailed description below.

### Example 1: Treatment of subepidermal blistering disorders

Pemphigus affects people across racial and cultural lines. It produces bum-like lesions that will not heal, which results of the loss of intercellular adhesion between the keratinocytes leading to bulla (blister) formation (Sharpe, R. J. in Manual of Clinical Problems in Dermatology, Olbricht, Bigby and Arndt eds., Little Brown & Co., Boston, 1992, pp. 56-60). Pemphigus vulgaris and Pemphigus vegetans are characterized by the formation of blisters above the basal layer of the skin. In Pemphigus foliaceus and Pemphigus erythematosus, blisters are observed just below the stratum corneum. For a review, see Ruocco E, et al, Precautions and suggestions for pemphigus patients, Dermatology 2001;203(3):201-7 and Hertl M, Veldman C, Pemphigus - paradigm of autoantibody-mediated autoimmunity, Int J Fertil Womens Med 2001 Jul-Aug;46(4):190-205.

Current treatments of pemphigus includes corticosteroids and immunosuppressive agents such as cyclophosphamide, azathioprine, methotrexate and cyclosporine-A (Lever, J. Am. Acad. Dermatol. 1979, Vol. 1, pp. 2-31). But, the severity of symptoms and the high mortality associated with pemphigus often lead to hospitalization. In addition, clinically significant bone loss occurs in the vast majority of patients exposed to corticosteroids with a very high risk for vertebral fracture, see Adachi JD, Corticosteroid-induced osteoporosis, Acta Derm Venereol 1999 Sep;79(5):351-5.

Bullous pemphigoid is more prevalent in elderly patients and include large tense blisters, on erythematous or non-erythematous skin or on urticarial plaques. A mortality rate of 10 to 20 percent is reported for the disease, largely due to side-effects from the use of systemic steroid therapy.

Cicatricial Pemphigoid involves primarily the mucous membranes (Baden, L. A., Manual of Clinical Problems in Dermatology, Little, Brown & Co., Boston, 1992, pp. 54). In many cases, this disorder involves desquamative gingivitis and ultimately leads to blindness. Current treatments are as mentioned above and are not satisfactory (Bleicher, supra; Arndt, K. in Fitzpatrick, Eisen, Wolff, Freedberg and Austen, Dermatology in General Medicine, 1987, Vol. 1, McGraw-Hill, Inc., New York, pp. 582-584). Antibiotics can also be used in combinaison with high dose corticosteroïds.

A common feature of pemphigus, bullous pemphigoid, cicatricial pemphigoid is the role of proteases in their pathogenesis (Grando, Glukhenky, Drannik, Kostromin and Chemyavsky, Int. J. Tissue React. 1989, Vol. 11, pp. 195-201). This diseases are classified as being mediated by proteases which affect especially the skin and mucosal membranes. In this regard, some proteinase inhibitors have been proposed in the treatment of pemphigus. Furthermore, in US 5,637,616, systemic administration of N-acetylcysteine is proposed for treating these diseases and in US 5,514,714 the use hypericin or pseudohypericin is described for treating pemphigus.

Still, as of today, none of the above available treatments are effective and safe for treating subepidermal blistering disorders. In addition, the prolonged use of immunosuppressor drugs lead to adverse side effects and morbidity.

A long time ago, participation of mast cells was suggested by a sequence of pathologic alterations in which there was progressive mast-cell degranulation and late eosinophil infiltration, Wintroub BU et al, Morphologic and functional evidence for release of mast-cell products in bullous pemphigoid, N Engl J Med 1978 Feb 23;298(8):417-21. More recently, a significant alterations in mast cell chymase and protease in different bullous diseases has been observed, suggesting mast cell involvement. But, it was thought that this reflected a general inflammation rather than a specific reaction, Kaminska R et al, Mast cells in developing subepidermal bullous diseases: emphasis on tryptase, chymase and protease inhibitors, Acta Derm Venereol 1999 Sep;79(5):351-5.

To stop such tissue degradation of skin and mucosal membranes, the present invention proposes to deplete mast cells using compounds that are substantially specific to mast cells. In this regard, c-kit specific kinase inhibitors are proposed to inhibit mast cell proliferation, survival and activation. Evidence of focal and complete degranulation of mast cells was observed in blisters or bullae of patients affected with pemphigus. Besides, it is was observed that B lymphocyte clones produce antibodies directed to the basal membrane of the epidermis. Here, we propose that activation of such detrimental immune response to the self can result from degranulation of mast cells. In addition, this activation of components of immunity goes with the release of proteases that further contribute to the degradation of tissues.

## Claims

1. Use of N-phenyl-2-pyrimidine-amine derivatives having the formula II : 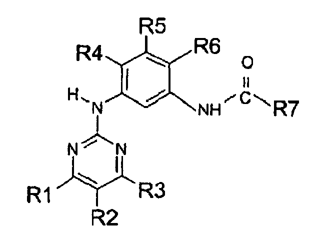 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group;
for preparing a medicament for treating autoimmune diseases selected from the group consisting of subepidermal blistering disorders, systemic lupus erythematosus, discoid lupus erythematosus, cutaneous lupus, dermatomyositis, polymyositis, Sjogren's syndrome, primary biliary cirrhosis, active chronic hepatitis, chronic fatigue syndrome and Vasculitis.

2. The use according to claim 1, wherein R7 is

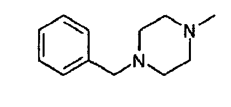

3. The use according to claim 2, wherein said inhibitor is the 4-(4-methylpiperazine-1-ylmethyl)-N-[4-methyl-3-(4-pyridine-3-yl)pyrimidine-2 ylamino)phenyl]-benzamide.

4. The use according to one of claims 1 to 3 for treating active chronic hepatitis and chronic fatigue syndrome.

5. The use according to one of claims 1 to 3 for treating for treating Lupus erythematosis.

6. The use according to one of claims 1 to 3 for treating subepidermal blistering disorders including aphthous ulcers, and several bullous diseases such as Pemphigus vulgaris, Pemphigus vegetans, Pemphigus foliaceus, and Pemphigus erythematosus, bullous pemphigoid and cicatricial pemphigoid.

7. The use according to one of claims 1 to 3 for treating Vasculitis.

8. The use of a composition suitable for topical administration comprising a c-kit inhibitor as defined in one of claims 1 to 3 for the treatment systemic lupus erythematosus, discoid lupus erythematosus, cutaneous lupus, dermatomyositis and Vasculitis.

9. The use of a c-kit inhibitor as defined in one of claims 1 to 3 combined with at least one antibiotic, preferably selected from dapsone, azathioprine, erythromycin, propionylerythromycin, neomycin, gentomycin, tobramycin, and mechlocycline for preparing a medicament for simultaneous, separate or sequential use for the treatment of subepidermal blistering disorders, such as pemphigus.

## Patentansprüche

1. Verwendung von N-Phenyl-2-pyrimidinamin-Derivaten mit der Formel II: 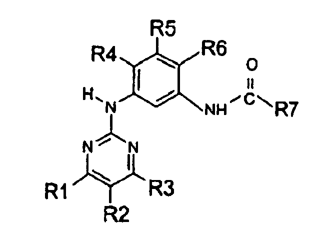 wobei, R1, R2 und R3 unabhängig ausgewählt sind aus H, F, Cl, Br, I und einem C1-C5-Alkyl oder einer cyclischen oder heterocyclischen Gruppe, insbesondere einer Pyridylgruppe;
R4, R5 und R6 unabhängig ausgewählt sind aus H, F, Cl, Br, I und einem C1-C5-Alkyl, insbesondere einer Methylgruppe;
und R7 eine Phenylgruppe ist, die wenigstens einen Substituenten aufweist, der wiederum wenigstens eine Aminogruppe besitzt,
zur Herstellung eines Arzneimittels zum Behandeln von Autoimmunkrankheiten ausgewählt aus der Gruppe bestehend aus Krankheiten mit subepidermaler Blasenbildung, systemischem Lupus erythematodes, diskoidem Lupus erythematodes, kutanem Lupus, Dermatomyositis, Polymyositis, Sjögren-Syndrom, primärer biliärer Leberzirrhose, aktiver chronischer Leberentzündung, chronischem Müdigkeitssyndrom und Vaskulitis.

2. Verwendung nach Anspruch 1, wobei R7 ist:

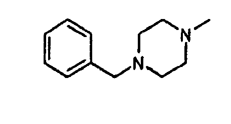

3. Verwendung nach Anspruch 2, wobei der Hemmer das 4-(4-Methylpiperazin-1-ylmethyl)-N-[4-methyl-3-(4-pyridin-3-yl)pyrimidin-2ylamino)phenyl]-benzamid ist.

4. Verwendung nach einem der Ansprüche 1 bis 3 zur Behandlung von aktiver chronischer Leberentzündung und chronischem Müdigkeitssyndrom.

5. Verwendung nach einem der Ansprüche 1 bis 3 zur Behandlung von Lupus erythematodes.

6. Verwendung nach einem der Ansprüche 1 bis 3 zur Behandlung von Krankheiten mit subepidermaler Blasenbildung, umfassend aphthöses Geschwür, und schweren Hautblasenerkrankungen, wie beispielsweise Pemphigus vulgaris, Pemphigus vegetans, Pemphigus foliaccus und Pemphigus erythematodes, bullösem Pemphigoid und Narbenpemphigoid.

7. Verwendung nach einem der Ansprüche 1 bis 3 zur Behandlung von Vaskulitis.

8. Verwendung einer Zusammensetzung, die zur topischen Verabreichung geeignet ist und einen c-Kit-Hemmer nach einem der Ansprüche 1 bis 3 umfasst, zur Behandlung von systemischem Lupus erythematodes, diskoidem Lupus erythematodes, kutanem Lupus, Dermatomyositis und Vaskulitis.

9. Verwendung eines c-Kit-Hemmers nach einem der Ansprüche 1 bis 3 in Kombination mit wenigstens einem Antibiotikum, das vorzugsweise aus Dapson, Azathioprin, Erythromycin, Propionylerythromycin, Neomycin, Gentomycin, Tobramycin und Mechlocyclin ausgewählt ist, für die Herstellung eines Arzneimittels zur gleichzeitigen, getrennten oder aufeinander folgenden Verwendung für die Behandlung von Krankheiten mit subepidermaler Blasenbildung, wie beispielsweise Pemphigus.

## Revendications

1. Utilisation de dérivés de N-phényl-2-pyrimidine-amines de formule II : 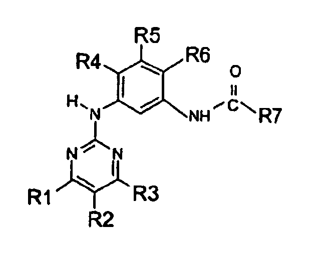 dans laquelle R1, R2 et R3 sont indépendamment choisis parmi H, F, Cl, Br, I, un radical alkyle en C1-C5 ou un groupe cyclique ou hétérocyclique, en particulier un groupe pyridyle ;
R4, R5 et R6 sont indépendamment choisis parmi H, F, Cl, Br, I, un radical alkyle en C1-C5, en particulier un groupe méthyle ;
et R7 est un groupe phényle portant au moins un substituant, qui à son tour possède au moins un groupe amino ;
pour la préparation d'un médicament destiné au traitement de maladies auto-immunes choisies dans le groupe constitué par les troubles bulleux sous-épidermiques, le lupus érythémateux disséminé, le lupus érythémateux chronique, le lupus cutané, la dermatomyosite, la polymyosite, le syndrome de Sjogren, la cirrhose biliaire primitive, l'hépatite chronique active, le syndrome de fatigue chronique, et l'angéite.

2. Utilisation selon la revendication 1, dans laquelle R7 est

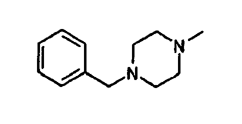

3. Utilisation selon la revendication 2, dans laquelle ledit inhibiteur est le 4-(4-méthylpipérazin-1-ylméthyl)-N-[4-méthyl-3-(4-pyridin-3-yl)pyrimidin-2-ylamino)phényl]benzamide.

4. Utilisation selon l'une des revendications 1 à 3, pour le traitement de l'hépatite chronique active et du syndrome de fatigue chronique.

5. Utilisation selon l'une des revendications 1 à 3, pour le traitement du lupus érythémateux.

6. Utilisation selon l'une des revendications 1 à 3, pour le traitement de troubles bulleux sous-épidermiques, y compris les ulcérations aphteuses, et de plusieurs maladies bulleuses telles que le pemphigus vulgaire, le pemphigus végétant, le pemphigus foliacé, et le pemphigus érythémateux, la pemphigoïde bulleuse, et la pemphigoïde cicatricielle.

7. Utilisation selon l'une des revendications 1 à 3, pour le traitement d'une angéite.

8. Utilisation d'une composition convenant pour une administration par voie topique comprenant un inhibiteur de c-kit tel que défini dans l'une des revendications 1 à 3, pour le traitement du lupus érythémateux disséminé, du lupus érythémateux chronique, du lupus cutané, de la dermatomyosite et de l'angéite.

9. Utilisation d'un inhibiteur de c-kit tel que défini dans l'une des revendications 1 à 3 en combinaison avec au moins un antibiotique, de préférence choisi parmi la dapsone, l'azathioprine, l'érythromycine, la propionylérythromycine, la néomycine, la gentomycine, la tobramycine, et la mechlocycline, pour la préparation d'un médicament destiné à une utilisation simultanée, séparée ou successive pour le traitement de troubles bulleux sous-épidermiques, tels qu'un pemphigus.
